# EUROPEAN PATENT SPECIFICATION

(11) **EP 3 419 439 B1**
(45) Date of publication and mention of the grant of the patent: **08.04.2020**
(21) Application number: 17706459.9
(22) Date of filing: 21.02.2017
(51) Int. Cl.: A23L 33/18, A23L 33/155, A23L 15/00

(54) **COMPOSITION FOR THE PREVENTION OR TREATMENT OF NEURODEGENERATIVE DISEASES**
ZUSAMMENSETZUNG ZUR PRÄVENTION ODER BEHANDLUNG VON NEURODEGENERATIVEN ERKRANKUNGEN
COMPOSITION POUR LA PRÉVENTION OU LE TRAITEMENT DE MALADIES NEURODÉGÉNÉRATIVES

(30) Priority: 22.02.2016 EP 16156649
(43) Date of publication of application: 02.01.2019
(73) Proprietor: Newtricious B.V., 5223 DE 's Hertogenbosch (NL)
(72) Inventor: JONKER, Paul Leopold, 5341 PX Oss (NL); VAN DER MADE, Sanne Maria, 5213 RG 'S-Hertogenbosch (NL); STERKMAN, Lucas Gerardus Willibrordus, 1852 RM Heiloo (NL)
(74) Representative: Algemeen Octrooi- en Merkenbureau B.V.
(86) International application number: PCT/EP2017/053891
(87) International publication number: WO 2017/144443

(56) References cited:
- EP-A1- 1 685 764
- EP-A1- 2 433 640
- WO-A1-2014/187942
- WO-A1-2015/113987
- US-A1- 2008 221 023
- US-A1- 2010 166 859
- US-A1- 2011 015 277
- HASEGAWA M ET AL: "Blood cholesterol lowering agent for preventing arterio sclerosis when mixed with food stuffs, feed and pharmaceuticals - consists of enzymatically digested product of ovomucin", WPI/THOMSON,, vol. 2000, no. 14, 30 June 1998 (1998-06-30), XP002528906,

## Description

### Field of the invention

The invention is in the field of the prevention, amelioration and treatment of neurodegenerative diseases, in particular of dementia, including the vascular form of dementia and Alzheimer's disease, Huntington's Disease, Parkinson's disease, Multiple Sclerosis and Amyotrophic lateral sclerosis. The invention provides new compositions that allow an improved prevention, amelioration and treatment of such diseases.

### Background of the invention

Neurodegeneration is a term for a range of overlapping conditions that primarily affect neurons in the human brain. These conditions are currently incurable and lead to the progressive loss of structure and/or function of neurons, which includes death of these cells. They result from failure in brain connectivity, which is formed by neuronal-neuronal, neuronal-glial, and glial-glial contacts. Neurons are the building blocks of the nervous system, which includes the brain and spinal cord. Neurons do not reproduce or replace themselves, so when they become damaged or die they cannot be replaced by the body. Neurodegenerative diseases (ND) cause problems with movement (ataxia) or mental functioning (dementia). Examples of neurodegenerative diseases are amyotrophic lateral sclerosis (ALS), Parkinson's disease, Huntington's disease, and dementia, which is most commonly known as Alzheimer's disease (AD). Dementias are responsible for the greatest burden of disease with Alzheimer's representing approximately 60-70% of cases. Generally, the risk of developing a neurodegenerative disease increases with aging.

The WHO has estimated that there are worldwide about 35 million AD patients; these numbers are expected to double by 2030 and triple by 2050 (1), which makes AD the most common neurodegenerative disease. Additionally, the impact of neurodegenerative diseases, such as AD, on health, quality of life, and health care costs shows the importance of finding preventive interventions that slow down the progression of ND. If prevention of cognitive decline -in the case of AD- is possible, we will be able to lower the risk of future disease and concomitant secondary damage at later ages.

The process of neurodegeneration has several aspects, which are not well-understood. Examples of these aspects are genetic mutations, protein misfolding, protein degradation and mitochondrial dysfunction. Although the understanding of neurodegenerative diseases has noticeable advanced in the past decades, well-established treatment and prevention measures are not available.

WO2014/187942 describes the treatment or prevention of neurodegenerative disorders using menthol, linalool and/or icilin. Currently, several drugs are marketed for the treatment of ND. These drugs claim to help delaying or preventing symptoms from becoming worse, but only for a limited time. Additionally, they may help control some behavioral symptoms. However, these drugs present themselves with various side effects such as nausea, vomiting, diarrhea, muscle cramps, fatigue, weight loss, dizziness, decreased appetite, constipation and headache. This shows the clear need for treatments that may intervene with multiple mechanisms of the development of neurodegeneration.

There are a number of animal models available to study the mechanisms and causes of neurodegenerative diseases. These animal models are widely used to study the efficacy of drugs for the treatment and prevention of neurodegenerative diseases. One of the best known models is the burrowing test, wherein the behavior of mice is observed in order to assess brain damage or malfunction as well as the progression of neurodegenerative diseases. The test seems particularly useful to detect early signs of beginning dysfunction and for the monitoring of the disease progression [30]. The model has been shown to be sensitive to hippocampus damage and the progression of neurodegenerative diseases (2). In at least one Alzheimer's disease model, neuropathological changes found in, amongst others, hippocampus regions, could be correlated with a significant reduction in burrowing performance (3).

### Summary of the invention.

Employing a mouse model for neurodegenerative diseases, we found that a composition, preferably an aqueous composition comprising a xanthophyll in combination with a hydrolysate of a protein comprising di- and tripeptides may advantageously be used to treat, prevent, or ameliorate neurodegenerative diseases.

### Detailed description of the invention.

The results described herein show that a composition comprising a xanthophyll and a hydrolysate of a protein, comprising di- and tripeptides provides a therapeutic effect on burrowing performance when administered to a test animal. The invention pertains to the subject-matter of the claims.

As used herein, the term "a xanthophyll" encompasses one or more species of xanthophylls and is equivalent to the term "at least one xanthophyll". Examples of suitable xanthophylls are lutein and zeaxanthin.

As used herein, the term "may" encompasses the word "can," and the term "may be" encompasses the words "is" or "are," depending on context. Furthermore, presence of the word "may" is intended to explain options for practicing or implementing the disclosure, without limitation.

As used herein, the term "a hydrolysate of a protein comprising di-and tripeptides" refers to a hydrolysate of a protein wherein the hydrolysate comprises a certain amount of di- and tripeptides that are derived from the protein as a consequence of hydrolysis.

As used herein, the term "hydrolysis" refers to the process in which a molecule of water is added to a substance. Such a reaction is preferably performed in the presence of an enzyme.

The composition showed a pronounced increase in 2h burrowing performance of LDLr-/- Leiden mice, and the effect of the composition was found to be synergistic, i.e. more than the sum of the effects of the xanthophyll and the hydrolysate separately.

There is ample evidence that xanthophylls have a beneficial effect on inflammatory processes in brain (4), skin (5), eye (6) and liver (7, 8). Xanthophylls may be conveniently administered to a subject in need of such a treatment.

In a preferred embodiment, the xantophyll may be contained in egg yolk. When chickens are fed with a diet enriched with xanthophylls, the yolk contains increased amounts of these natural substances, which are found in the micelles. However, the amounts of xanthophylls that can be safely administered are on the one hand limited by the amount of xanthophylls contained in the egg yolk and on the other hand by the maximum amount of egg yolk that can be safely administered to a subject. In order to maximize the amount of xanthophylls that can be effectively delivered in the blood stream, strategies have been deployed to maximize the absorption of xanthophylls in the gut.

It has previously been described that the absorption in the gut may be greatly enhanced by mixing the xanthophylls-containing egg yolk with polar lipids (21). For example certain dairy products are good sources of suitable polar lipids or phospholipids.

The egg yolk may be formulated as an aqueous dispersion, such as a dairy dispersion. The absorption in the gut of xanthophylls such as lutein and
zeaxanthin is greatly enhanced by this formulation (9, 10). Without wanting to be bound by theory, it is thought that this improved absorption is due to the formation of micelles that are present in the mixtures (11).

An aqueous dispersion comprising dairy products, such as buttermilk and egg yolk containing lutein and zeaxanthin, has been used to treat individuals with early signs of age-related macular degeneration, and has shown a positive effect on visual acuity (21).

Protein hydrolysates containing di- and tripeptides have been used to treat diseases as well. A salmon protein hydrolysate has been shown to decrease the expression of ICAM-1, VCAM-1 and MCP-1 in the aortic arch of apoE-/- mice (12). *In vitro* research showed the ability of an almond protein hydrolysate to modulate levels of IL-6, IL-1β and TNF-α in macrophages (13).

EP 1685764 A1 describes the use of a food product comprising a protein hydrolysate selected from ovomucin, lysozyme and ovotransferrin for treating high blood pressure.

In Zucker Diabetic Fatty rats, a dose of 1-3 gram per day of a hydrolysate of lysozyme from egg white showed effects on inflammatory markers (14). It has also been shown that a hydrolysate of lysozyme from egg white reduced renal interleukin (II)-1b/II-13 mRNA expression, renal tumor necrosis factor (TNF)-α, mRNA and P22phox protein expression and glomerulosclerosis. The same composition additionally reduced albuminuria, and restored aortic endothelium-dependent relaxation (EDR). Indomethacin added to the organ bath instantly improved aortic EDR, indicating a role for cyclo-oxygenase (COX)-derived contractile prostanoids in opposing relaxation in ZDF rats. This indomethacin effect was reduced by a hydrolysate of lysozyme from egg white, and coincided with decreased renal COX-1/2 protein expression. Thus, protein hydrolysates comprising di- and tripeptides were shown to have anti-inflammatory effects. Effects on neurodegeneration have not been shown so far.

We have now found that a composition comprising a xanthophyll and a hydrolysate of a protein comprising di- and tripeptides may be advantageously used in the treatment, amelioration or prevention of neurodegenerative diseases.

The hydrolysate comprising di- and tripeptides is preferably obtained by digesting a protein with a hydrolyzing enzyme. The protein is selected
from the group consisting of lysozyme, ovomucin, ovotransferrin, ovalbumin and casein. The hydrolyzing
enzyme is preferably an endopeptidase, such as a serine protease. In a particularly advantageous embodiment, the serine protease is a subtilase, preferably subtilisin, more preferably Alcalase™.

The composition according to the invention may further comprise an additional pharmacologically active or nutritionally beneficial compound, such as a compound selected from the group consisting of an omega-3 fatty acid, docosahexaenoic acid, eicosapentaenoic acid, Uridin, vitamin D, Folic acid, Vitamin E, xanthophylls, iodine, selenium and zinc. In one embodiment, the composition according to the invention is an aqueous composition.

It is particularly preferred that the content of di- and tripeptides is above 5% of the total protein content of the composition according to the invention. In a preferred embodiment, the di- and tri-peptides may make up at least 10% of the total protein content of the composition.

It is even more preferred that at least 30%, such as 40% or 45%, such as at least 50%, 60%, 70%, 75%, 80%, 85%, 90%, 95% or even at least 98% of the peptides in the composition, such as the aqueous composition have a molecular weight below 500 Da. This fraction contains the di- and tripeptides.

There are a large number of methods available for determining the total protein content in a food product. The skilled person is well aware of the pros and cons of each of these methods and will be able to choose an appropriate method depending on the choice of the food product. Just by way of example, the well-known Kjeldahl method may be used with ovalbumin as the standard.

The composition according to the invention may be a ready-for-use solution, as a stock solution from which the daily dose may be obtained or prepared by dilution or it may be a dry composition from which a daily dose may be obtained by adding a fluid. The composition may also be used as dry matter and mixed with a food stuff, The skilled person is well aware of these and other ways of administering a composition to a subject in need of the composition.

The di-and tripeptide content may also be expressed as a percentage of the total protein content of the composition. The composition according to the invention may therefore also be characterized by the feature that at least 30% of the peptides in the hydrolysate comprising di- and tripeptides have a molecular weight of less than 0.5 kD.

In a preferred embodiment, the composition comprises at least 10 gram of di- and tripeptides per kg of composition, such as at least 20, 40, 60, 80 or 100 gram per kg of composition. In a further preferred embodiment, the composition comprises at least 200, 400, 800, or added up to 1000 gram of di- and tripeptides per kg of composition.

A suitable daily dose of the composition for a human is between 5 and 250 gram, preferably between 10 and 200 gram, such as between 20 and 100 gram, such as 25 gram, or 50 gram. A skilled person is well aware of the recommended daily dose suitable for other subjects such as non-human animals.

One daily dose should preferably contain about at least 500 mg of di-and tripeptides, such as at least 1000 mg, 2000 mg or 5000 mg (Table 1).

**Table 1: Preferred compositions according to the invention**

| **Ingredient:** | **Preferred ready for use [mg/kg composition]** | **Recommended daily dose [mg]** | **Preferred minimum conc. [mg/kg composition]** | **Preferred maximum conc. [mg/kg composition]** |
|---|---|---|---|---|
| Dairy polar lipids | 500 | 12 | 20 | 10.000 |
| Xanthophyll | 50 | 1 | 5 | 500 |
| Omega-3 fatty acid | 9.000 | 200 | 1.000 | 50.000 |
| Di- and tripeptides | 100.000 | 2.000 | 10.000 | 900.000 |

The xanthophyll may be contained in an aqueous or non-aqueous solution or in a dry form. It is however preferred that the xanthophyll is contained in an aqueous dispersion. Such a dispersion is contained in the term "aqueous composition" and may be selected from the group consisting of skimmed milk, semi-skimmed milk, buttermilk, a buttermilk fraction, fermented milk, yoghurt, soy drink, soy milk, fermented soy milk, fruit juices, fruit purees, syrups, vegetable juices and vegetable purees. Preferably, the aqueous dispersion is buttermilk or a buttermilk fraction.

In a preferred embodiment, a composition according to the invention comprises at least 20 mg of dairy polar lipids per kg of composition, such as at least 40, 60, 80, or 100 mg/kg. In a preferred embodiment, the composition comprises at least 200 mg of dairy polar lipids, such as 300, 400, 500, 600, 700, 800 or 900 mg per kg of composition. Whereas there is hardly any upper limit for the dairy polar lipid content of the composition, for practical purposes the dairy polar lipid content may be kept below 10.000 mg per kg of the composition.

A xanthophyll content of at least 2 mg per kg of composition is preferred, preferably the composition comprises at least 3, 4, 5, 6, 8, 10, 15, 20, 30 or even at least 50 mg per kg. Preferred xanthophylls are lutein and zeaxanthin. In a further preferred embodiment, the composition comprises at least 10 mg lutein per kg composition, such as 12, 14, 16, 18 or at least 20 mg per kg, such as 25, 30, 35 or at least 40 mg per kg.

In a further preferred embodiment, the invention relates to a composition comprising at least 1000 mg of omega-3 fatty acids such as DHA per kg composition, such as at least 2000. 3000. 4000, 5000, 6000, 7000, 8000, 9000 or even 10000 mg per kg composition or more. Although there is no maximum for the DHA content of the composition, for practical purposes the composition may not contain more than 50000 mg of DHA per kg of composition.

A preferred daily dose of the omega-3 fatty acid is about 50 mg, such as 100 mg, such as 150 mg, 200, 400 or 500 mg.

It should be noted that the term 'buttermilk' as used herein refers to a dairy product obtained in a fermentation of a dairy product, such as whole milk. The use of what may be called 'synthetic buttermilk', such as acidified low fat milk or fat free milk is less preferred, although it is often labelled as buttermilk by dairy manufacturers. The fermentation process enriches buttermilk with the desirable polar lipids, which are present at only low levels or entirely absent in the synthetic buttermilk products. Generally, dairy products such as buttermilk, containing at least 80 mg of polar lipids per liter are suitable for use in the manufacture of the composition of the present invention. Preferred is the use of dairy products with an even higher dairy polar lipid content, such as at least 100 mg per liter, such as 120, 140, 180. 220, 260, 300, 340, 380 or even at least 420 mg per liter.

The xanthophyll is preferably selected from the group consisting of zeaxanthin, lutein and meso-zeaxanthin and may be comprised in egg yolk. So the composition according to the invention may comprise egg yolk or an egg yolk fraction containing the xanthophyll component of the egg yolk.

It is particularly preferred that the egg yolk and the aqueous dispersion are present in a weight ratio between 1:2 to 1:7, particularly preferred are ratios between 1:2 and 1:3.

The compositions as described herein may also be in a concentrated, dehydrated or dry form, obtainable by dehydrating the aqueous compositions as described herein.

The composition according to the invention may advantageously be employed as a food product, as a food supplement, or as a medicament for the treatment of a disease. It is to be used in the treatment, prevention or amelioration of a neurodegenerative disease, selected from the group consisting of dementia, Alzheimer's disease, Huntington's Disease, Parkinson's disease, Multiple Sclerosis and Amyotrophic Lateral Sclerosis.

### Examples

### Example 1: production of a composition comprising a xanthophyll.

A composition comprising a xanthophyll was produced from eggs obtained by feeding lutein and zeaxanthin to chickens and collecting the yolks from eggs produced by these chickens (WO 2009/078716). These eggs are referred to as enriched eggs herein.

Feeds for producing the enriched eggs were formulated and produced within the legal requirements for animal feed. The use of lutein and zeaxanthin is regulated under EU regulation 1831/2003. The dosage of lutein and zeaxanthin in feed did not exceed the legal limit of 80 ppm in animal feed.

Enriched eggs produced by poultry that were fed the lutein and zeaxanthin enriched feed contained 45 - 80 mg lutein per kg egg yolk and 10-30 mg zeaxanthin per kg egg yolk.

The animals were also fed a diet rich in omega-3 fatty acids; the eggs contained approximately 200 mg omega-3 fatty acids per kg egg yolk, with a standard deviation of 10 mg.

### Example 2 : Manufacturing of an aqueous dispersion comprising xanthophylls.

Eggs enriched with xanthophylls and DHA were produced under an ISO 22000/HACCP certified quality scheme as described in example 1. Eggs were separated in yolk and albumen in an automated facility in an ISO 22000:2005 certified plant. Egg yolk from 165,000 enriched eggs was mixed (15 min., 4°C) with 5,500 liters of buttermilk containing 80 mg polar lipids per liter, and 170 kg of sugar. The liquid was pasteurized for 3 minutes at 65°C and cooled to 4°C. This composition is herein referred to as NWT-02 or NWT-02 liquid formulation.

For storage, the composition was dried to a powder with 4(±1)% moisture content and mixed with free flowing agent (SiO2, Sipernat 22S).

### Example 3: Preparation of a protein hydrolysate comprising di- and tripeptides.

A 5% (w/v) solution of lysozyme in water (100% protein content, Belovo SA, Bastogne, Belgium) was prepared and adjusted to a pH between 7.5 and 8.5 with 3M KOH. Hydrolysis was started by adding Alcalase^{(Tm)} (Novozymes) to a final concentration of 4% on protein basis. The solution was incubated for a total of 5-6 hours at 60°C, under continuous stirring. Alcalase was then inactivated by increasing the temperature to 90°C for 15 minutes. The solution was then cooled down to 2°C and stored overnight under continuous stirring.

The resulting hydrolysate solution was filtered through a 10µm filter and subsequently through a 1µm filter. Thereafter, the filtrate was heat treated for 15s at 135°C and concentrated to a dry matter of 57°Brix (approximately dry matter of 45%) by a NIRO evaporator at a flow of 3300L/h at 90°C. After evaporation, the product was spray dried to obtain a powder with very good flowability properties, as evidenced by visual observation.

The final product had the following characteristics: white powder, good solubility, degree of hydrolysis of 21% (15) and a maximum molecular weight of less than 10 kDa. Peptide size distribution was as follows: 46% <500Da, 23% 500-1000 Da, 32% >1000 Da. This product is herein further referred to as NWT-03.

### Example 4: Preparation of alternative protein hydrolysates.

Ovomucin, ovotransferrin, ovalbumin and casein were each individually hydrolysed by a mixture of four different commercially available proteases (protease mixtures, Newlase F, Promod 278P, Alcalase and Umamizyme).

The proteins were dissolved in water and incubated with the enzyme mix according to the manufacturer's instructions. The enzymes were then inactivated by increasing the temperature to 90°C for 15 minutes. The solution was then cooled down to 2°C and stored overnight under continuous stirring.

The hydrolysate solution was filtered through a 10 µm filter and subsequently through a 1µm filter. Thereafter, the filtrate was heat treated for 15s at 135°C and concentrated to a dry matter of 57°Brix by a NIRO evaporator at a flow of 3300L/h at 90°C. After evaporation, the product was spray dried to obtain a powder with very good flowability properties, as evidenced by visual observation.

The final product had the following characteristics: white powder, good solubility, degree of hydrolysis of 24% and a maximum molecular weight of less than 10 kDa. Peptide size distribution was as follows: 98% <500Da, 1% 500-1000 Da, 1% >1000 Da.

### Example 5: Preparation of NWT-02 for mice diets.

NWT-02 was fed to mice as ad-mix through a high-fat diet, comprising 59 g NWT02/kg high fat diet containing 24% (w/w) lard fat (Research Diets, D12541, USA). This amounts to an intake of 0.16 g NWT-02/mouse/day which equals an intake of between 7 and 9 micrograms lutein/mouse/day.

### Example 6: Preparation of NWT-03 for mice diets.

NWT-03 was fed to mice as ad-mix through a high-fat diet, comprising 35.7 g NWT03/kg high fat diet containing 24% (w/w) lard fat (Research Diets, D12541, USA). This amounts to an intake of 0.1 g NWT03/mouse/day.

### Example 7: Preparation of NWT-02 and NWT-03 in mice diets.

NWT-02 plus NWT-03 was fed to mice as ad-mix through a high-fat diet, comprising 59 g NWT02/kg high fat diet and 35.7 g NWT03/kg high fat diet containing 24% (w/w) lard fat (Research Diets, D12541, USA). This amounts to an intake of 0.16 g NWT-02 plus 0.1 g NWT-03/mouse/day.

### Example 8: In vivo animal experiments.

The study was performed using 48 male, approx. 12 weeks old, LDLr-/- Leiden mice. Mice were fed a high fat diet (HFD) containing 24% lard, as described and used earlier (16) with or without the NWT-02 and/or NWT-03 preparations as described above. All mice were fed a HFD from t=0 until t=9 weeks. Mice were matched at t=9 weeks into 4 groups of 12 mice based on body weight and plasma glucose levels. The groups formed were:
1) HFD control (n=12)
2) HFD + NWT-02 (n=12)
3) HFD + NWT-03 (n=12)
4) HFD + NWT-02 +NWT-03 (n=12)

### Example 9: Functional tests

At t=9 and t=21 weeks, the mice were tested in a burrowing test as follows (according to Deacon, J Vis Exp. 2012 Jan 5; (59):e2607).

Mice were habituated to the procedure a week prior to the test by placement of the burrow tube into the home cage. After two baseline measurements (overnight, 48 h apart), the burrow test was performed. The mouse was placed in a cage with the burrow tube containing 200 g of food pellets. The tube was weighed after 2 hours.

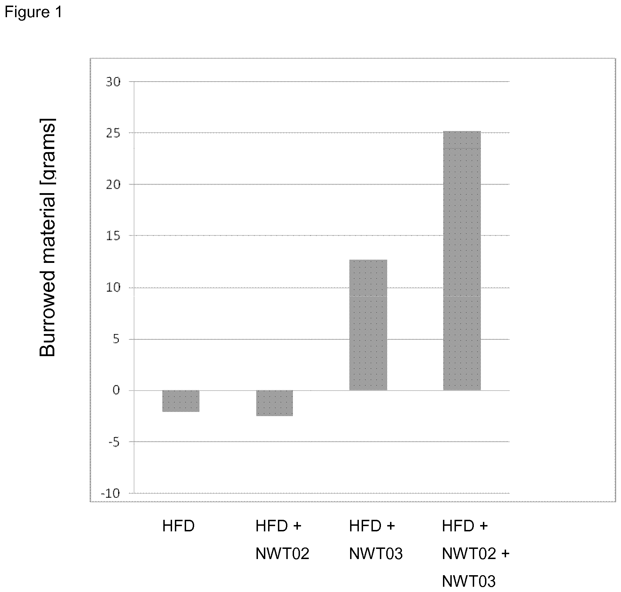

The difference between the burrowing behaviour at the start (t = 9 weeks) and the end (t = 21 weeks) of the treatment was determined. It was found that the mice fed with a high fat diet (group 1) and those fed with a high fat diet in combination with NWT-02 burrowed substantially the same amount of material (-2,1 gram and -2,5 gram respectively) at the beginning and the end of the test (figure 1).

Mice fed with a high fat diet containing NWT-03 burrowed 12,7 gram on average, whereas the mice fed with a high fat diet comprising both NWT-02 and NWT-03 burrowed 25,2 grams on average.

It is concluded that NWT-03 has a positive effect on cognition and may be used for the treatment of neurodegenerative diseases. This effect is synergistically enhanced by the addition of NWT-02.

### Example 10; testing of equivalent NWT03 preparations

The functional tests as described in example 9 were repeated with alternative sources of di- and tripeptides obtained by enzymatic digestion of Ovomucin, ovotransferrin, ovalbumin and casein, as described in example 4. The difference observed in the burrowing behaviour were marginal; all preparations tested showed that the protein hydrolysates had a positive effect on cognition and may therefore be used for the treatment of neurodegenerative diseases. This effect was again synergistically enhanced by the addition of NWT-02.

### REFERENCES

1. Libby P. Inflammation in atherosclerosis. Nature. 2002 Dec 19-26;420(6917):868-74. PubMed PMID: 12490960.
2. Telander DG. Inflammation and age-related macular degeneration (AMD). Seminars in ophthalmology. 2011 May;26(3):192-7. PubMed PMID: 21609232.
3. Wyss-Coray T. Inflammation in Alzheimer disease: driving force, bystander or beneficial response? Nature medicine. 2006 Sep;12(9):1005-15. PubMed PMID: 16960575.
4. Sullivan F. Smart Prevention - Health Care Cost Savings Resulting from the Targeted Use of Dietary Supplements. 2013.
5. Nolan JM. Personal communication. Waterford University; 2013.
6. Kershaw EE, Flier JS. Adipose tissue as an endocrine organ. The Journal of clinical endocrinology and metabolism. 2004 Jun;89(6):2548-56. PubMed PMID: 15181022.
7. Bastard JP, Jardel C, Bruckert E, Blondy P, Capeau J, Laville M, et al. Elevated levels of interleukin 6 are reduced in serum and subcutaneous adipose tissue of obese women after weight loss. The Journal of clinical endocrinology and metabolism. 2000 Sep;85(9):3338-42. PubMed PMID: 10999830.
8. Mohamed-Ali V, Flower L, Sethi J, Hotamisligil G, Gray R, Humphries SE, et al. beta-Adrenergic regulation of IL-6 release from adipose tissue: in vivo and in vitro studies. The Journal of clinical endocrinology and metabolism. 2001 Dec;86(12):5864-9. PubMed PMID: 11739453.
9. Loffreda S, Yang SQ, Lin HZ, Karp CL, Brengman ML, Wang DJ, et al. Leptin regulates proinflammatory immune responses. FASEB journal : official publication of the Federation of American Societies for Experimental Biology. 1998 Jan;12(1):57-65. PubMed PMID: 9438411.
10. Esposito K, Nappo F, Marfella R, Giugliano G, Giugliano F, Ciotola M, et al. Inflammatory cytokine concentrations are acutely increased by hyperglycemia in humans: role of oxidative stress. Circulation. 2002 Oct 15;106(16):2067-72. PubMed PMID: 12379575.
11. Petersen AM, Pedersen BK. The anti-inflammatory effect of exercise. Journal of applied physiology. 2005 Apr;98(4):1154-62. PubMed PMID: 15772055.
12. Rogowski O, Shapira I, Bassat OK, Chundadze T, Finn T, Berliner S, et al. Waist circumference as the predominant contributor to the micro-inflammatory response in the metabolic syndrome: a cross sectional study. Journal of inflammation. 2010;7:35. PubMed PMID: 20659330. Pubmed Central PMCID: 2919526.
13. Mohamed-Ali V, Goodrick S, Bulmer K, Holly JM, Yudkin JS, Coppack SW. Production of soluble tumor necrosis factor receptors by human subcutaneous adipose tissue in vivo. The American journal of physiology. 1999 Dec;277(6 Pt 1):E971-5. PubMed PMID: 10600783.
14. Clement K, Viguerie N, Poitou C, Carette C, Pelloux V, Curat CA, et al. Weight loss regulates inflammation-related genes in white adipose tissue of obese subjects. FASEB journal: official publication of the Federation of American Societies for Experimental Biology. 2004 Nov;18(14):1657-69. PubMed PMID: 15522911.
15. Stitzinger M. Lipids, inflammation and atherosclerosis. Leiden: Leiden/Amsterdam Center for Drug Researh; 2007.
16. Li SY, Yang D, Fu ZJ, Woo T, Wong D, Lo AC. Lutein enhances survival and reduces neuronal damage in a mouse model of ischemic stroke. Neurobiology of disease. 2012 Jan;45(1):624-32. PubMed PMID: 22024715.
17. Evans JA, Johnson EJ. The role of phytonutrients in skin health. Nutrients. 2010 Aug;2(8):903-28. PubMed PMID: 22254062. Pubmed Central PMCID: 3257702.
18. Kijlstra A, Tian Y, Kelly ER, Berendschot TT. Lutein: more than just a filter for blue light. Progress in retinal and eye research. 2012 Jul;31(4):303-15. PubMed PMID: 22465791.
19. Kim JE, Clark RM, Park Y, Lee J, Fernandez ML. Lutein decreases oxidative stress and inflammation in liver and eyes of guinea pigs fed a hypercholesterolemic diet. Nutrition research and practice. 2012 Apr;6(2):113-9. PubMed PMID: 22586499. Pubmed Central PMCID: 3349032.
20. Meriwether LS, Humphrey BD, Peterson DG, Klasing KC, Koutsos EA. Lutein exposure, in ovo or in the diet, reduces parameters of inflammation in the liver and spleen laying-type chicks (Gallus gallus domesticus). Journal of animal physiology and animal nutrition. 2010 Oct;94(5):e115-22. PubMed PMID: 20546071.
21. WO2009/078716; Thielen WJG, Berendschot TTJM, Nelissen JWPM, Plat J, inventors. Method of producing egg yolk based functional food product and products obtainable thereby.
22. Chung HY, Rasmussen HM, Johnson EJ. Lutein bioavailability is higher from lutein-enriched eggs than from supplements and spinach in men. The Journal of nutrition. 2004 Aug;134(8):1887-93. PubMed PMID: 15284371.
23. Kelly ER, Plat J, Haenen GR, Kijlstra A, Berendschot TT. The effect of modified eggs and an egg-yolk based beverage on serum lutein and zeaxanthin concentrations and macular pigment optical density: results from a randomized trial. PloS one. 2014;9(3):e92659. PubMed PMID: 24675775. Pubmed Central PMCID: 3968018.
24. Abdel-Aal el SM, Akhtar H, Zaheer K, Ali R. Dietary sources of lutein and zeaxanthin carotenoids and their role in eye health. Nutrients. 2013 Apr;5(4):1169-85. PubMed PMID: 23571649. Pubmed Central PMCID: 3705341.
25. Parolini C, Vik R, Busnelli M, Bjorndal B, Holm S, Brattelid T, et al. A salmon protein hydrolysate exerts lipid-independent anti-atherosclerotic activity in ApoE-deficient mice. PloS one. 2014;9(5):e97598. PubMed PMID: 24840793. Pubmed Central PMCID: 4026378.
26. Udenigwe CC, Je JY, Cho YS, Yada RY. Almond protein hydrolysate fraction modulates the expression of proinflammatory cytokines and enzymes in activated macrophages. Food & function. 2013 Apr 30;4(5):777-83. PubMed PMID: 23575976.
27. Wang Y, Landheer S, van Gilst WH, van Amerongen A, Hammes HP, Henning RH, et al. Attenuation of renovascular damage in Zucker diabetic fatty rat by NWT-03, an egg protein hydrolysate with ACE- and DPP4-inhibitory Activity. PloS one. 2012;7(10):e46781. PubMed PMID: 23071636. Pubmed Central PMCID: 3468629.
28. Radonjic M, Wielinga PY, Wopereis S, Kelder T, Goelela VS, Verschuren L, et al. Differential effects of drug interventions and dietary lifestyle in developing type 2 diabetes and complications: a systems biology analysis in LDLr-/- mice. PloS one. 2013;8(2):e56122. PubMed PMID: 23457508. Pubmed Central PMCID: 3574110.
29. van der Made SM, Kelly ER, Berendschot TT, Kijlstra A, Lutjohann D, Plat J. Consuming a Buttermilk Drink Containing Lutein-Enriched Egg Yolk Daily for 1 Year Increased Plasma Lutein but Did Not Affect Serum Lipid or Lipoprotein Concentrations in Adults with Early Signs of Age-Related Macular Degeneration. The Journal of nutrition. 2014 Jul 2. PubMed PMID: 24991045.
30. Jirkof, P. Burrowing and nest building behavior as indicators of well-being in mice. J. Neuroscience Methods 234 (2014) 139-146.

## Claims

1. An aqueous composition comprising:
a. at least one xanthophyll and
b. a hydrolysate of a protein, comprising di- and tripeptides
for use in the treatment, prevention or amelioration of a neurodegenerative disease, wherein the neurodegenerative disease is selected from the group consisting of dementia, Alzheimer's disease, Huntington's Disease, Parkinson's disease, Multiple Sclerosis and Amyotrophic Lateral Sclerosis, wherein the protein is selected from the group consisting of lysozyme, ovomucin, ovotransferrin, ovalbumin and casein.

2. The composition for use according to claim 1, wherein the hydrolysate is obtainable by treating the protein with an enzyme.

3. The composition for use according to claim 2, wherein the enzyme is an endopeptidase.

4. The composition for use according to claim 2 wherein the enzyme is selected from the group of enzymes consisting of a serine protease, subtilase, subtilisin, and Alcalase™.

5. The composition for use according to any one of claims 1 - 4, wherein the composition additionally comprises a pharmacologically active compound selected from the group consisting of an omega-3 fatty acid, docosahexaenoic acid, eicosapentaenoic acid, vitamin D, Uridin, Folic acid, Vitamin E, iodine, selenium and zinc.

6. The composition for use according to any one of claims 1 - 5 wherein the di- and tri-peptides make up at least 10% of the total protein content of the composition.

7. The composition for use according to any one of claims 1 - 6 wherein the di- and tripeptides have a molecular weight of less than 0.5 kD.

8. The composition for use according to any one of claims 1 - 7 wherein the at least one xanthophyll is contained in an aqueous dispersion.

9. The composition for use according to claim 8 wherein the aqueous dispersion is selected from the group consisting of skimmed milk, semi-skimmed milk, buttermilk, a buttermilk fraction, fermented milk, yoghurt, soy drink, soy milk, fermented soy milk, fruit juices, fruit purees, syrups, vegetable juices and vegetable purees.

10. The composition for use according to claim 9 wherein the aqueous dispersion is buttermilk or a buttermilk fraction.

11. The composition for use according to any one of claims 1 - 10 wherein the at least one xanthophyll is selected from the group consisting of zeaxanthin, lutein and meso-zeaxanthin.

12. The composition for use according to any one of claims 1 - 11 additionally comprising egg yolk.

13. The composition for use according to claim 12 wherein the egg yolk and the aqueous dispersion are present in a weight ratio between 1:2 to 1:7, preferably between 1:2 and 1:3.

14. A composition for use according to any one of claims 1 - 13 wherein the aqueous composition is dehydrated.

15. A composition for use according to any one of claims 1 - 14 wherein the aqueous composition is contained in a food product.

## Patentansprüche

1. Eine wässrige Zusammensetzung, umfassend:
a. mindestens ein Xanthophyll und
b. ein Hydrolysat eines Proteins, das Di- und Tripeptide zur Verwendung bei
der Behandlung, Vorbeugung oder Verbesserung einer neurodegenerativen Erkrankung umfasst, worin die neurodegenerative Erkrankung ausgewählt ist aus der Gruppe bestehend aus Demenz, Alzheimer-Krankheit, Huntington-Krankheit, Parkinson-Krankheit, Multipler Sklerose und Amyotropher Lateralsklerose, worin das Protein ausgewählt ist aus der Gruppe bestehend aus Lysozym, Ovomucin, Ovotransferrin, Ovalbumin und Kasein.

2. Die Zusammensetzung zur Verwendung nach Anspruch 1, worin das Hydrolysat durch Behandeln des Proteins mit einem Enzym erhältlich ist.

3. Die Zusammensetzung zur Verwendung nach Anspruch 2, worin das Enzym eine Endopeptidase ist.

4. Die Zusammensetzung zur Verwendung nach Anspruch 2, worin das Enzym ausgewählt ist aus der Gruppe von Enzymen, bestehend aus Serinprotease, Subtilase, Subtilisin und Alcalase™.

5. Die Zusammensetzung zur Verwendung nach einem der Ansprüche 1 - 4, worin die Zusammensetzung zusätzlich eine pharmakologisch aktive Verbindung enthält, ausgewählt aus der Gruppe bestehend aus einer Omega-3-Fettsäure, Docosahexaensäure, Eicosapentaensäure, Vitamin D, Uridin, Folsäure, Vitamin E, Jod, Selen und Zink.

6. Die Zusammensetzung zur Verwendung nach einem der Ansprüche 1 - 5, worin die Di- und Tri-Peptide mindestens 10% des Gesamtproteingehalts der Zusammensetzung ausmachen.

7. Die Zusammensetzung zur Verwendung nach einem der Ansprüche 1 - 6, worin die Di- und Tripeptide ein Molekulargewicht von weniger als 0,5 kD aufweisen.

8. Die Zusammensetzung zur Verwendung nach einem der Ansprüche 1 - 7, worin das mindestens eine Xanthophyll in einer wässrigen Dispersion enthalten ist.

9. Die Zusammensetzung zur Verwendung nach Anspruch 8, worin die wässrige Dispersion ausgewählt ist aus der Gruppe bestehend aus Magermilch, teilentrahmter Milch, Buttermilch, einer Buttermilchfraktion, fermentierter Milch, Joghurt, Sojagetränk, Sojamilch, fermentierter Sojamilch, Fruchtsäften, Fruchtpürees, Sirupen, Gemüsesäften und Gemüsepürees.

10. Die Zusammensetzung zur Verwendung nach Anspruch 9, worin die wässrige Dispersion Buttermilch oder eine Buttermilchfraktion ist.

11. Die Zusammensetzung zur Verwendung nach einem der Ansprüche 1 - 10, worin das mindestens eine Xanthophyll aus der Gruppe bestehend aus Zeaxanthin, Lutein und Meso-Zeaxanthin ausgewählt ist.

12. Die Zusammensetzung zur Verwendung nach einem der Ansprüche 1 - 11, ferner umfassend Eigelb.

13. Die Zusammensetzung zur Verwendung nach Anspruch 12, worin das Eigelb und die wässrige Dispersion in einem Gewichtsverhältnis zwischen 1:2 bis 1:7, vorzugsweise zwischen 1:2 und 1:3, vorliegen.

14. Eine Zusammensetzung zur Verwendung nach einem der Ansprüche 1 -13, wobei die wässrige Zusammensetzung dehydratisiert ist.

15. Eine Zusammensetzung zur Verwendung nach einem der Ansprüche 1 - 14, wobei die wässrige Zusammensetzung in einem Nahrungsmittelprodukt enthalten ist.

## Revendications

1. Composition aqueuse comprenant :
a. au moins une xanthophylle, et
b. un hydrolysat d'une protéine, comprenant des di- et tripeptides,
pour utilisation dans le traitement, la prévention ou l'amélioration d'une maladie neurodégénérative, où la maladie neurodégénérative est choisie dans le groupe constitué de la démence, de la maladie d'Alzheimer, de la maladie de Huntington, de la maladie de Parkinson, de la Sclérose en Plaques et de la Sclérose Latérale Amyotrophique, où la protéine est choisie dans le groupe constitué du lysozyme, de l'ovomucine, de l'ovotransferrine, de l'ovalbumine et de la caséine.

2. Composition pour utilisation selon la revendication 1, dans laquelle l'hydrolysat peut être obtenu en traitant la protéine avec une enzyme.

3. Composition pour utilisation selon la revendication 2, dans laquelle l'enzyme est une endopeptidase.

4. Composition pour utilisation selon la revendication 2, dans laquelle l'enzyme est choisie dans le groupe d'enzymes constitué d'une sérine protéase, d'une subtilase, d'une subtilisine et d'une Alcalase™.

5. Composition pour utilisation selon l'une quelconque des revendications 1 à 4, dans laquelle la composition comprend en outre un composé pharmacologiquement actif choisi dans le groupe constitué d'un acide gras oméga-3, de l'acide docosahexaénoïque, de l'acide eicosapentaénoïque, de la vitamine D, de l'uridine, de l'acide folique, de la vitamine E, de l'iode, du sélénium et du zinc.

6. Composition pour utilisation selon l'une quelconque des revendications 1 à 5, dans laquelle les di- et tripeptides constituent au moins 10% de la teneur totale en protéines de la composition.

7. Composition pour utilisation selon l'une quelconque des revendications 1 à 6, dans laquelle les di- et tripeptides ont un poids moléculaire inférieur à 0,5 kD.

8. Composition pour utilisation selon l'une quelconque des revendications 1 à 7, dans laquelle l'au moins une xanthophylle est contenue dans une dispersion aqueuse.

9. Composition pour utilisation selon la revendication 8, dans laquelle la dispersion aqueuse est choisie dans le groupe constitué du lait écrémé, du lait demi-écrémé, du babeurre, d'une fraction de babeurre, du lait fermenté, du yaourt, d'une boisson de soja, du lait de soja, du lait de soja fermenté, des jus de fruits, des purées de fruits, des sirops, des jus de légumes et des purées de légumes.

10. Composition pour utilisation selon la revendication 9, dans laquelle la dispersion aqueuse est du babeurre ou une fraction de babeurre.

11. Composition pour utilisation selon l'une quelconque des revendications 1 à 10, dans laquelle l'au moins une xanthophylle est choisie dans le groupe constitué de la zéaxanthine, de la lutéine et de la méso-zéaxanthine.

12. Composition pour utilisation selon l'une quelconque des revendications 1 à 11, comprenant en outre du jaune d'œuf.

13. Composition pour utilisation selon la revendication 12, dans laquelle le jaune d'œuf et la dispersion aqueuse sont présents selon un rapport pondéral compris entre 1 : 2 et 1 : 7, de préférence entre 1 : 2 et 1 : 3.

14. Composition pour utilisation selon l'une quelconque des revendications 1 à 13, dans laquelle la composition aqueuse est déshydratée.

15. Composition pour utilisation selon l'une quelconque des revendications 1 à 14, dans laquelle la composition aqueuse est contenue dans un produit alimentaire.
